# EUROPÄISCHE PATENTSCHRIFT

(11) **EP 4 389 740 B1**
(45) Veröffentlichungstag und Bekanntmachung des Hinweises auf die Patenterteilung: **30.04.2025**
(21) Anmeldenummer: 22215281.1
(22) Anmeldetag: 21.12.2022
(51) Int. Cl.: C07D 319/12

(54) **POLYMERISIERBARES TETRAMETHYLGLYCOLID**
POLYMERIZABLE TETRAMETHYLGLYCOLIDE
TÉTRAMÉTHYLGLYCOLID POLYMÉRISABLE

(43) Veröffentlichungstag der Anmeldung: 26.06.2024
(73) Patentinhaber: Röhm GmbH, 64295 Darmstadt (DE)
(72) Erfinder: VORHOLZ, Johannes, 63755 Alzenau (DE); WEBER, Andreas, 60596 Frankfurt (DE); TRESKOW, Marcel, 64293 Darmstadt (DE); KRILL, Steffen, 64367 Mühltal (DE); DENGER, Marcus, 64395 Brensbach (DE); HOFFMANN, Norbert, 64347 Griesheim (DE); KLEIN, Isabelle, 63801 Kleinostheim (DE); PAPADOPOULOS, Nikolaos, 65428 Rüsselsheim (DE); SCHNABEL, Michael, 64584 Biebesheim (DE)
(74) Vertreter: Röhm Patent Association

(56) Entgegenhaltungen:
- WO-A1-95/09142
- US-A1- 2010 010 276

## Beschreibung

### Gebiet der Erfindung

Die vorliegende Erfindung betrifft ein neues Verfahren zur Herstellung von polymerisierbarem Tetramethylglycolid. In diesem Verfahren wird zunächst eine Reaktionsmischung, die 2-Hydroxyisobuttersäure enthält, umgesetzt unter Erhalt einer Produktmischung, die Tetramethylglycolid enthält. Dieses wird anschließend kristallisiert und abgetrennt unter Erhalt des polymerisierbaren Tetramethylglycolids. Darüber hinaus betrifft die vorliegende Erfindung das durch das erfindungsgemäße Verfahren erhältliche polymerisierbare Tetramethylglycolid.

### Stand der Technik

Poly(2-Hydroxyisobuttersäure) (Poly(2-HIBS)) wurde erstmals 1922 von Blaise und Montagne synthetisiert (Blaise, E. E., Montagne M., A. Compte Rendu 174 (1922) 1553). In deren Herstellverfahren wurde als Monomerbaustein 2-Hydroxyisobuttersäure-Anhydrosulfit eingesetzt. Ein derartiges Homopolymer ist auch in der US 2,811,511 offenbart.

Deibig, H., Geiger, J., Sander, M., Makromol. Chem. 145 (1971) 121, Deibig, H., Geiger, J., Sander, M., Makromol. Chem. 145 (1971) 133, Nishida, H., Andou, Y., Watanabe, K, Arazoe, Y., Ide, S., Shirai, Y., Macromol. 44 (2011) 12 und Watanabe, K., Andou, Y., Shirai, Y., Nishida, H., Chem. Lett. 42 (2013) 159 beschreiben jeweils die Herstellung von Poly(2-HIBS) ausgehend von 2-Hydroxyisobuttersäure (2-HIBS). Die Polymerisation erfolgt jeweils durch ringöffnende Polymerisation von Tetramethylglycolid (TMG), dem cyclischen Dimer der 2-Hydroxyisobuttersäure. Das erhaltene Poly(2-HIBS) zeichnet sich durch Transparenz, gute mechanische Eigenschaften, hohe chemische Beständigkeit und eine geringe Neigung zur Kristallisation aus. Zudem können daraus dauerhaft flexible Filme hergestellt werde. Auch ist Poly(2-HIBS) vollständig rezyklierbar.

Im Gegensatz dazu schließen Hall, H. K., Schneider, A. K., J. Am. Chem. Soc 80 (1958) 6409, Brandrup, J., Immergut, E. H., Grulke, E.A., (Editors) Polymer Handbook, 4th Edition, Volume 1 (1999) II/404, Salyasombat, W., Molloy, R., Nicholson, T. M., Johnson, A. F., Ward, I. M., Poshyachinda, B., Polymer, 39 (1998) 5581, und Kricheldorf, H., Lomadze, N., Schwarz, G., J. Polym. Sci. A 46 (2008) 6229 eine Polymerisation von 2-HIBS aus.

Die WO 2008/061821 betrifft die Herstellung von TMG durch Erhitzen von 2-HIBS auf eine Temperatur von mindestens 100 °C. Ebenso beschreibt die EP 0 834 511 A ein Verfahren zur Herstellung von cyclischen Estern durch Depolymerisation von Polymeren oder durch Cyclisierung von α-Hydroxycarbonsäuren.

Die im Stand der Technik beschriebenen cyclischen Ester, insbesondere TMG, weisen häufig eine nicht ausreichende Reinheit auf, um aus ihnen Polymere herzustellen. Zudem sind die im Stand der Technik beschriebenen Verfahren zur Polymerisation von cyclischen Estern und zur Herstellung von polymerisierbaren cyclischen Estern nicht zuverlässig reproduzierbar. US 2010/010276 A1 offenbart ein Verfahren zur Herstellung von Tetramethylglycolid, umfassend das Erhitzen einer Zusammensetzung, die mindestens 50 Gew.-% 2-Hydroxyisobuttersäure und Tetramethylglycolid umfasst, auf eine Temperatur von mindestens 150°C bei einem Druck von 0,1 bis 0,4 bar, wobei die Reaktion autokatalytisch ausgeführt wird. WO 95/09142 A1 betrifft ein Verfahren zur Herstellung von zyklischen Estern durch die Umwandlung von Hydroxycarbonsäuren und ihren Derivaten in ihre jeweiligen zyklischen Ester und offenbart die Herstellung des zyklischen Diesters von α-Hydroxyisobuttersäure (TMG).

Es besteht daher Bedarf an Verfahren, um cyclische Ester, insbesondere TMG, mit besonders hoher Reinheit darzustellen. Insbesondere sollen die cyclischen Ester ausreichend rein sein, um Polymere daraus herzustellen und die Polymerisation der cyclischen Ester soll zuverlässig reproduzierbar sein.

### Aufgabe

Die der vorliegenden Erfindung zugrundeliegende Aufgabe bestand darin, ein Verfahren bereitzustellen, das die Herstellung von Tetramethylglycolid mit besonders hoher Reinheit erlaubt. Insbesondere sollte das so erhältliche Tetramethylglycolid zur Polymerisation, insbesondere zur Herstellung von Poly(2-Hydroxyisobuttersäure), geeignet sein.

### Lösung

Gelöst wurde diese Aufgabe durch ein Verfahren zur Herstellung von polymerisierbarem Tetramethylglycolid umfassend die folgenden Schritte a) bis c):
a) Umsetzen einer Reaktionsmischung, die 2-Hydroxyisobuttersäure enthält, in einem ersten Reaktor bei einer ersten Temperatur, die im Bereich von 150 °C bis 200 °C liegt, und einem ersten Druck, der im Bereich von 100 mbar bis 1013 mbar liegt, wobei der Umsatzgrad der 2-Hydroxyisobuttersäure bei der Umsetzung bei maximal 95 % liegt, unter Erhalt einer Produktmischung, die
   5 bis 50 Gew.-% 2-Hydroxyisobuttersäure,
   25 bis 85 Gew.-% Tetramethylglycolid und
   5 bis 25 Gew.-% Di-2-Hydroxyisobuttersäure enthält, jeweils bezogen auf das Gesamtgewicht der Produktmischung,
b) Kristallisation des in der Produktmischung enthaltenen Tetramethylglycolids unter Erhalt von kristallisiertem Tetramethylglycolid und einer Produktflüssigphase,
c) Abtrennung des in Schritt b) kristallisierten Tetramethylglycolids von der Produktflüssigphase unter Erhalt des polymerisierbaren Tetramethylglycolids.

Gelöst wurde diese Aufgabe weiterhin durch polymerisierbares Tetramethylglycolid erhältlich nach dem erfindungsgemäßen Verfahren.

Das erfindungsgemäße Verfahren erlaubt überraschenderweise die Herstellung von besonders reinem Tetramethylglycolid (TMG), das zudem zu Poly(2-Hydroxyisobuttersäure) polymerisierbar ist.

Das erfindungsgemäße Verfahren erlaubt insbesondere die Herstellung von Tetramethylglycolid ausgehend von Acetoncyanhydrin-Herstellverfahren, insbesondere aus einem Acetoncyanhydrin-Herstellverfahren, bei dem Aceton mit Blausäure zu Acetoncyanhydrin umgesetzt wird.

Die durch Polymerisation des mit dem erfindungsgemäßen Verfahren hergestellten TMG erhältliche Poly(2-Hydroxyisobuttersäure) zeichnet sich in vorteilhafter Weise durch hohe Transparenz und vollständige biologische Abbaubarkeit zu CO₂ aus. Poly(2-Hydroxyisobuttersäure) kann zudem durch herkömmliche Verfahren wie Spritzguss, Gussverfahren, Extrusionsverfahren oder Verfahren zur Herstellung von Fasern oder Folien verarbeitet werden und ist in ihre Ausgangsmaterialien, 2-Hydroxyisobuttersäure und TMG, spaltbar.

Poly(2-Hydroxyisobuttersäure) zeichnet sich zudem durch eine höhere Hydrolysebeständigkeit und Glastemperatur im Vergleich zu Poly(Milchsäure) aus, was die Verarbeitung und Verwendung in Bereichen ermöglicht, in denen Poly(Milchsäure) nicht verwendet werden kann.

Nachfolgend wird das erfindungsgemäße Verfahren näher beschrieben.

In Schritt a) des erfindungsgemäßen Verfahrens wird eine Reaktionsmischung in einem ersten Reaktor bei einer ersten Temperatur und einem ersten Druck umgesetzt unter Erhalt einer Produktmischung.

Die Reaktionsmischung enthält 2-Hydroxyisobuttersäure. 2-Hydroxyisobuttersäure ist als solche bekannt und hat die CAS-Nummer 594-61-6. Sie wird auch als 2-Hydroxy-2-methylpropansäure oder 2-HIBS bezeichnet. Nachfolgend werden daher die Begriffe "2-Hydroxyisobuttersäure" und "2-HIBS" synonym gebraucht und besitzen die gleiche Bedeutung.

Die Reaktionsmischung kann darüber hinaus mindestens eine weitere Komponente enthalten. Die mindestens eine weitere Komponente ist beispielsweise ausgewählt aus der Gruppe bestehend aus Tetramethylglycolid, Di-2-Hydroxyisobuttersäure und oligomerer 2-Hydroxyisobuttersäure.

Bevorzugt ist daher auch ein Verfahren, bei dem die Reaktionsmischung zusätzlich mindestens eine weitere Komponente ausgewählt aus der Gruppe bestehend aus Tetramethylglycolid, Di-2-Hydroxyisobuttersäure und oligomere 2-Hydroxyisobuttersäure enthält.

Die mindestens eine weitere Komponente ist beispielsweise in der Reaktionsmischung enthalten, wenn in einer bevorzugten Ausführungsform der Erfindung der in Schritt a1) erhaltene erste Kopfstrom in Schritt a) rückgeführt wird und/oder die in Schritt c) erhaltene Produktflüssigphase in Schritt a) rückgeführt wird.

Die Reaktionsmischung ist bevorzugt erhältlich durch ein Verfahren zur Herstellung von Acetoncyanhydrin durch Umsetzung von Aceton mit Blausäure.

Bevorzugt ist daher auch ein Verfahren, bei dem die in Schritt a) umgesetzte Reaktionsmischung erhältlich ist durch ein Verfahren zur Herstellung von Acetoncyanhydrin durch Umsetzung von Aceton mit Blausäure.

Verfahren zur Herstellung von Acetoncyanhydrin durch Umsetzung von Aceton mit Blausäure sind als solche bekannt.

Bei diesem Verfahren wird üblicherweise Aceton mit Blausäure umgesetzt unter Erhalt von Acetoncyanhydrin. Dieses kann mit Schwefelsäure weiter umgesetzt werden zu Sulfoxy-alpha-Hydroxyisobuttersäureamid. Dieses wird auch als SIBA bezeichnet. SIBA kann dann bei Temperaturen beispielsweise im Bereich von 90 °C bis 120 °C in Gegenwart von Wasser zu 2-Hydroxyisobuttersäure und Ammoniumsulfat umgesetzt werden. Dieses Verfahren ist als solches bekannt.

Die so erhaltene 2-Hydroxyisobuttersäure kann von Ammoniumsulfat beispielsweise mittels Destillation und/oder Extraktion abgetrennt werden und dann als Reaktionsmischung in Schritt a) umgesetzt werden. Eine so erhältliche Reaktionsmischung enthält üblicherweise noch Reste von Ammoniumsulfat.

Insbesondere wenn die Reaktionsmischung durch ein Verfahren zur Herstellung von Acetoncyanhydrin durch Umsetzung von Aceton mit Blausäure erhältlich ist, enthält die Reaktionsmischung also zusätzlich Ammoniumsulfat.

Der Umsatzgrad der 2-Hydroxyisobuttersäure bei der Umsetzung liegt bei maximal 95 %, bevorzugt im Bereich von 50 % bis 90 %, mehr bevorzugt im Bereich von 60 % bis 85 % und am meisten bevorzugt im Bereich von 70 % bis 80 %. Unter dem Umsatzgrad der 2-Hydroxyisobuttersäure wird im Rahmen der vorliegenden Erfindung der Anteil an der umgesetzten Menge 2-Hydroxyisobuttersäure bezogen auf die vor Beginn der Umsetzung in der Reaktionsmischung enthaltene Menge an 2-Hydroxyisobuttersäure verstanden. Die Bestimmung kann nach dem Fachmann bekannten Methoden erfolgen, beispielsweise mittels HPLC oder GC.

Die Produktmischung enthält daher im Bereich von 5 bis 50 Gew.-% 2-Hydroxyisobuttersäure, bevorzugt im Bereich von 5 bis 10 Gew.-%, jeweils bezogen auf das Gesamtgewicht der Produktmischung.

Bei der Umsetzung in Schritt a) dimerisiert 2-HIBS. Es bildet sich zunächst durch Kondensation die weiter unten beschriebene Di-2-Hydroxyisobuttersäure. Aus Di-2-Hydroxyisobuttersäure bildet sich dann durch erneute Kondensation Tetramethylglycolid.

Tetramethylglycolid ist als solches bekannt und wird auch als 3,3,6,6-Tetramethyl-1,4-dioxan-2,5-dion bezeichnet. Ebenso ist es unter der Bezeichnung TMG bekannt. Die Begriffe "Tetramethylglycolid" und "TMG" werden daher im Rahmen der vorliegenden Erfindung synonym gebraucht und haben dieselbe Bedeutung. TMG ist das cyclische Dimer von 2-HIBS.

Die Produktmischung enthält im Bereich von 25 bis 85 Gew.-% TMG, bevorzugt im Bereich von 80 bis 85 Gew.-% TMG, jeweils bezogen auf das Gesamtgewicht der Produktmischung.

Bei der Dimerisierung von 2-HIBS bildet sich wie vorstehend beschrieben Di-2-Hydroxyisobuttersäure. Im Rahmen der vorliegenden Erfindung wird unter Di-2-Hydroxyisobuttersäure 2-((2-hydroxy-2-methylpropanoyl)oxy)-2-methylpropansäure verstanden. Di-2-Hydroxyisobuttersäure ist als solche bekannt und hat die CAS-Nummer 87422-65-9. Di-2-Hydroxyisobuttersäure wird auch als Di-2-HIBS bezeichnet. Im Rahmen der vorliegenden Erfindung werden daher die Begriffe "Di-2-Hydroxyisobuttersäure", "2-((2-hydroxy-2-methylpropanoyl)oxy)-2-methylpropansäure" und "Di-2-HIBS" synonym gebraucht und besitzen daher dieselbe Bedeutung.

Die Produktmischung enthält im Bereich von 5 bis 25 Gew.-% Di-2-HIBS, bevorzugt im Bereich von 10 bis 20 Gew.-% Di-2-HIBS, jeweils bezogen auf das Gesamtgewicht der Produktmischung.

Bei der Umsetzung der Reaktionsmischung oligomerisiert 2-HIBS außerdem üblicherweise teilweise. Bei der Oligomerisierung von 2-HIBS bilden sich beispielsweise Trimere, Tetramere und Pentamere von 2-HIBS. Oligomerisierte 2-HIBS wird im Rahmen der vorliegenden Erfindung auch als "oligomere 2-Hydroxyisobuttersäure" bezeichnet.

Die Produktmischung kann daher beispielsweise im Bereich von 0,1 bis 10 Gew.-%, bevorzugt im Bereich von 0,5 bis 5 Gew.-% oligomere 2-Hydroxyisobuttersäure enthalten, jeweils bezogen auf das Gesamtgewicht der Produktmischung.

Die Dimerisierung und die Oligomerisierung von 2-HIBS sind Kondensationsreaktionen. Daher wird bei der Dimerisierung und Oligomerisierung von 2-HIBS zudem Wasser gebildet. Daher enthält die Produktmischung üblicherweise zusätzlich Wasser.

Das bei der Umsetzung gebildete Wasser kann während der Umsetzung zumindest teilweise aus dem ersten Reaktor entfernt werden, beispielsweise durch die in einer bevorzugten Ausführungsform der Erfindung vom ersten Reaktor umfasste Kolonne. Die Entfernung des gebildeten Wassers ist vorteilhaft, da dadurch das Gleichgewicht der Umsetzung von 2-HIBS hin zu TMG verschoben wird.

Beispielsweise enthält die in Schritt a) erhaltene Produktmischung zusätzlich 0,001 bis 0,5 Gew.-% Wasser, bevorzugt 0,005 bis 0,2 Gew.-% Wasser, bezogen auf das Gesamtgewicht der Produktmischung.

Enthält die Reaktionsmischung zusätzlich Ammoniumsulfat und/oder andere stickstoffhaltige Verbindungen, so bilden sich bei der Umsetzung der Reaktionsmischung in Schritt a) beispielsweise zusätzlich 1,3-substituierte Diketone von Morpholin, die mit vier Methylgruppen substituiert sind, und/oder 1,4-substituierte Diketone von Morpholin, die mit vier Methylgruppen substituiert sind. Daher kann die Produktmischung zusätzlich 1,3-substituierte Diketone von Morpholin, die mit vier Methylgruppen substituiert sind, und/oder 1,4-substituierte Diketone von Morpholin, die mit vier Methylgruppen substituiert sind, enthalten.

Bei der Umsetzung der Reaktionsmischung in Schritt a) kann zudem in der Reaktionsmischung gegebenenfalls enthaltenes Tetramethylglycolid und/oder bei der Umsetzung gebildetes Tetramethylglycolid zumindest teilweise gespalten werden. Dabei bilden sich Methacrylsäure, Aceton und Kohlenmonooxid. Kohlenmonooxid wird üblicherweise als Gas aus der Umsetzung in Schritt a) entfernt. Daher kann die Produktmischung zusätzlich Methacrylsäure und/oder Aceton enthalten.

Die Umsetzung in Schritt a) kann über einen beliebigen Zeitraum stattfinden. Vorzugsweise liegt die Reaktionsdauer der Umsetzung in Schritt a) im Bereich von 1 Stunde bis 24 Stunden, besonders bevorzugt im Bereich von 6 Stunden bis 9 Stunden.

Bevorzugt ist daher auch ein Verfahren, bei dem die Reaktionsdauer der Umsetzung in Schritt a) im Bereich von 1 Stunde bis 24 Stunden liegt.

Unter der "Reaktionsdauer" wird der Zeitraum von Erreichen der ersten Temperatur bis zum Erreichen des Umsatzgrades der 2-Hydroxyisobuttersäure verstanden.

Die Umsetzung in Schritt a) kann in Batchfahrweise stattfinden, ebenso ist es möglich, dass sie kontinuierlich stattfindet. Für den Fall, dass die Umsetzung in Schritt a) kontinuierlich stattfindet, wird unter der Reaktionsdauer die mittlere Verweilzeit der Reaktionsmischung in dem ersten Reaktor verstanden.

Die Umsetzung kann in Gegenwart eines Katalysators stattfinden, bevorzugt in Gegenwart eines sauren Katalysators. Geeignete saure Katalysatoren zur Katalysierung der Umsetzung in Schritt a) sind als solche bekannt und beispielsweise ausgewählt aus der Gruppe bestehend aus anorganischen Säuren, organischen Säuren und sauren Kationentauschern. Geeignete anorganische Säuren sind beispielsweise Schwefelsäure oder Salzsäure. Geeignete organische Säuren sind beispielsweise Sulfonsäuren wie p-Toluolsulfonsäure. Darüber hinaus können beispielsweise in einer Polymermatrix eingebettete sulfonierte Aromaten als stark saure Ionentauscher eingesetzt werden. Ein derartiger in einer Polymermatrix eingebetteter sulfonierter Aromat ist beispielsweise sulfoniertes Polystyrol in einer Polystyrolmatrix.

Besonders bevorzugt dient 2-Hydroxyisobuttersäure selbst als saurer Katalysator bei der Umsetzung. Bevorzugt läuft die Umsetzung also autokatalytisch ab.

Bevorzugt ist daher auch ein Verfahren, bei die Umsetzung in Schritt a) autokatalytisch abläuft.

Die Umsetzung findet bei einer ersten Temperatur statt. Die erste Temperatur liegt im Bereich von 150 °C bis 200 °C, bevorzugt im Bereich von 170 °C bis 190 °C.

Die erste Temperatur bezieht sich auf die Temperatur im Inneren des ersten Reaktors. Dem Fachmann ist klar, dass, wenn der erste Reaktor in einer bevorzugten Ausführungsform eine Kolonne umfasst, die Temperatur in der Kolonne niedriger als die erste Temperatur sein kann.

Die Umsetzung findet bei einem ersten Druck statt. Der erste Druck liegt im Bereich von 100 mbar bis 1013 mbar, bevorzugt im Bereich von 200 mbar bis 400 mbar.

Die Umsetzung in Schritt a) findet in einem ersten Reaktor statt. Als erster Reaktor eignen sich dem Fachmann bekannte Reaktoren für Cyclisierungen, wie beispielsweise Rührkesselreaktoren. Ebenso kann als erster Reaktor eine Rührkesselkaskade eingesetzt werden. Dies ist inbesondere vorteilhaft, wenn das erfindungsgemäße Verfahren kontinuierlich durchgeführt werden soll. Als Reaktormaterial wird vorzugsweise Edelstahl oder Emaille verwendet.

Vorzugsweise umfasst der erste Reaktor mindestens eine Kolonne, besonders bevorzugt mindestens eine Destillationskolonne und/oder mindestens eine Rektifikationskolonne.

Bevorzugt ist daher auch ein Verfahren, bei dem der erste Reaktor mindestens eine Kolonne umfasst.

Über die Kolonne wird vorzugsweise bei der Umsetzung gebildetes Wasser zumindest teilweise entfernt. Wasser kann zumindest teilweise als Azeotrop mit 2-HIBS entfernt werden. Ebenso kann über die Kolonne beispielsweise bei der Spaltung von Tetramethylglycolid gebildetes Aceton entfernt werden.

In Schritt b) des erfindungsgemäßen Verfahrens wird das in der Produktmischung enthaltene Tetramethylglycolid kristallisiert unter Erhalt von kristallisiertem Tetramethylglycolid und einer Produktflüssigphase.

Das in der Produktmischung enthaltene TMG kann nach dem Fachmann bekannten Methoden kristallisiert werden. Bevorzugt wird die Produktmischung zur Kristallisation des Tetramethylglycolids abgekühlt auf eine zweite Temperatur. Die zweite Temperatur liegt beispielsweise im Bereich von 50 °C bis 80 °C. Bevorzugt erfolgt die Kristallisation in Schritt b) also bei einer zweiten Temperatur im Bereich von 50 °C bis 80 °C.

Bevorzugt ist daher auch ein Verfahren, bei dem die Kristallisation in Schritt b) bei einer zweiten Temperatur im Bereich von 50 °C bis 80 °C stattfindet.

Es ist möglich, dass die Kristallisation mit einem ersten Lösungsmittel erfolgt. Beispielsweise kann ein erstes Lösungsmittel zu der Produktmischung gegeben werden und so TMG kristallisiert werden. Erfolgt die Kristallisation mit einem ersten Lösungsmittel, so kann die Produktmischung zusätzlich abgekühlt werden zur Kristallisation. Als erstes Lösungsmittel eignet sich beispielsweise ein Lösungsmittel ausgewählt aus der Gruppe bestehend aus Isopropanol, Methyl-iso-butylketon, Toluol, Pentan, Hexan, substituierten Hexan-Derivaten und cyclisierten Hexan-Derivaten.

Bevorzugt erfolgt die Kristallisation ohne Zugabe eines ersten Lösungsmittels zur Produktmischung.

Bevorzugt wird daher Tetramethylglycolid aus einer Schmelze der Produktmischung kristallisiert.

Insbesondere bevorzugt wird Tetramethylglycolid in Schritt b) fraktioniert kristallisiert. Verfahren zur fraktionierten Kristallisation sind als solche bekannt.

In Schritt c) des erfindungsgemäßen Verfahrens wird das in Schritt b) kristallisierte Tetramethylglycolid von der Produktflüssigphase abgetrennt unter Erhalt des polymerisierbaren Tetramethylglycolids.

Das in Schritt b) kristallisierte Tetramethylglycolid kann nach dem Fachmann bekannten Methoden von der Produktflüssigphase abgetrennt werden, beispielsweise mittels Filtration oder Zentrifugation. Das kristallisierte Tetramethylglycolid kann zur Abtrennung von der Produktflüssigphase zusätzlich gewaschen werden. Beispielsweise kann das kristallisierte Tetramethylglycolid zunächst filtriert und anschließend gewaschen werden. Zum Waschen eignen sich dem Fachmann bekannte zweite Lösungsmittel, in denen sich Tetramethylglycolid schwer löst. Derartige Lösungsmittel sind beispielsweise 2-Propanol, Methyl-iso-butylketon und/oder Ethanol.

Insbesondere wenn Tetramethylglycolid in Schritt b) fraktioniert kristallisiert wird, ist es bevorzugt, dass nach Abtrennung der Produktflüssigphase in Schritt c) das kristallisierte Tetramethylglycolid teilweise aufgeschmolzen wird. Dadurch werden Verunreinigungen aus dem kristallisierten Tetramethylglycolid entfernt, sodass dann polymerisierbares Tetramethylglycolid erhalten wird.

Bei der Abtrennung des kristallisierten Tetramethylglycolids von der Produktflüssigphase wird das polymerisierbare Tetramethylglycolid erhalten. Zudem wird die Produktflüssigphase erhalten. Die Produktflüssigphase enthält üblicherweise die Komponenten, die in der Produktmischung enthalten waren, und die nicht mit Tetramethylglycolid in Schritt b) kristallisiert wurden.

Beispielsweise enthält die Produktflüssigphase zumindest eine Komponente ausgewählt aus der Gruppe bestehend aus 2-Hydroxyisobuttersäure, Tetramethylglycolid, Di-2-Hydroxyisobuttersäure, oligomere 2-Hydroxyisiobuttersäure und Wasser.

Wurde Tetramethylglycolid in Schritt b) durch Zugabe eines ersten Lösungsmittels kristallisiert, so enthält die Produktflüssigphase üblicherweise zusätzlich dieses erste Lösungsmittel.

Bevorzugt wird die in Schritt c) erhaltene Produktflüssigphase in die Reaktionsmischung in Schritt a) rückgeführt.

Bevorzugt ist daher auch ein Verfahren, bei dem die in Schritt c) erhaltene Produktflüssigphase in Schritt a) rückgeführt wird.

Enthält die Produktflüssigphase zusätzlich ein erstes Lösungsmittels, so wird dieses vorzugsweise vor Rückführung der Produktflüssigphase in die Reaktionsmischung abgetrennt.

Verfahren zur Abtrennung des ersten Lösungsmittels aus der Produktflüssigphase sind als solche bekannt. Beispielsweise kann das erste Lösungsmittel durch Destillation abgetrennt werden, ebenso ist eine Abtrennung durch Phasentrennung möglich. Bevorzugt wird das erste Lösungsmittel durch Destillation abgetrennt.

Enthält die Produktmischung in einer vorstehend beschriebenen Ausführungsform oligomere 2-Hydroxyisobuttersäure, so enthält üblicherweise auch die in Schritt c) erhaltene Produktflüssigphase oligomere 2-Hydroxyisobuttersäure. Die oligomere 2-Hydroxyisobuttersäure wird bevorzugt vor Rückführung der Produktflüssigphase in Schritt a) aus der Produktflüssigphase abgetrennt.

Beispielsweise wird oligomere 2-Hydroxyisobuttersäure aus der Produktflüssigphase abgetrennt indem zur Produktflüssigphase ein Katalysator gegeben wird. Der Katalysator ist bevorzugt ein Zinn(IV)oxidkatalysator. Als Zinn(IV)oxidkatalysator eignet sich insbesondere Dioctylzinnoxid. Dioctylzinnoxid ist als solches bekannt und hat die CAS-Nummer 870-08-6. Es wird auch als DOTO bezeichnet.

Durch den Katalysator wird oligomere 2-Hydroxyisobuttersäure gespalten in kürzere Oligomere von 2-Hydroxyisobuttersäure, in 2-Hydroxyisobuttersäure, Di-2-Hydroxyisobuttersäure und/oder Tetramethylglycolid. Die bei der Spaltung gebildeten kürzeren Oligomere von 2-Hydroxyisobuttersäure werden üblicherweise durch den Katalysator weiter gespalten zu 2-Hydroxyisobuttersäure, Di-2-Hydroxyisobuttersäure und/oder Tetramethylglycolid. Bei der Spaltung gebildetes TMG kann von der Produktflüssigphase abdestilliert werden, die Produktflüssigphase, von der oligomere 2-Hydroxyisobuttersäure abgetrennt wurde, kann dann in Schritt a) rückgeführt werden.

Bevorzugt ist daher auch ein Verfahren, bei dem die in Schritt a) erhaltene Produktmischung zusätzlich oligomere 2-Hydroxyisobuttersäure enthält und diese in Gegenwart eines Zinn(IV)oxidkatalysators gespalten und in Schritt a) rückgeführt wird.

Bevorzugt wird im erfindungsgemäßen Verfahren im Anschluss an Schritt a) und vor Schritt b) der folgende Schritt a1) durchgeführt:
a1) Destillation der in Schritt a) erhaltenen Produktmischung bei einem zweiten Druck, der im Bereich von 100 mbar bis 500 mbar liegt, unter Erhalt eines ersten Sumpfstroms und eines ersten Kopfstroms, wobei der erste Sumpfstrom
mindestens 80 Gew.-% TMG,
höchstens 1 Gew.-% 2-HIBS,
höchstens 2 Gew.-% Di-2-Hydroxyisobuttersäure und
mindestens 5 Gew.-% oligomere 2-Hydroxyisobuttersäure enthält, jeweils bezogen **auf** das Gesamtgewicht des ersten Sumpfstroms,
und wobei der erste Kopfstrom
0,1 bis 5 Gew.-% Di-2-Hydroxyisobuttersäure,
10 bis 50 Gew.-% 2-Hydroxyisobuttersäure und
40 bis 85 Gew.-% Tetramethylglycolid enthält, jeweils bezogen auf das Gesamtgewicht des ersten Kopfstroms,
und in Schritt b) dann das in dem ersten Sumpfstrom enthaltene Tetramethylglycolid kristallisiert wird.

Bevorzugt ist daher auch ein Verfahren, bei dem im Anschluss an Schritt a) und vor Schritt b) der folgende Schritt a1) durchgeführt wird
a1) Destillation der in Schritt a) erhaltenen Produktmischung bei einem zweiten Druck, der im Bereich von 100 mbar bis 500 mbar liegt, unter Erhalt eines ersten Sumpfstroms und eines ersten Kopfstroms, wobei der erste Sumpfstrom
mindestens 80 Gew.-% Tetramethylglycolid,
höchstens 1 Gew.-% 2-Hydroxyisobuttersäure,
höchstens 2 Gew.-% Di-2-Hydroxyisobuttersäure und
mindestens 5 Gew.-% oligomere 2-Hydroxyisobuttersäure enthält, jeweils bezogen auf das Gesamtgewicht des ersten Sumpfstroms,
und wobei der erste Kopfstrom
0,1 bis 5 Gew.-% Di-2-Hydroxyisobuttersäure,
10 bis 50 Gew.-% 2-Hydroxyisobuttersäure und
40 bis 85 Gew.-% Tetramethylglycolid enthält, jeweils bezogen auf das Gesamtgewicht des ersten Kopfstroms,
und in Schritt b) dann das in dem ersten Sumpfstrom enthaltene Tetramethylglycolid kristallisiert wird.

Wird Schritt a1) durchgeführt, so wird also in Schritt b) das in dem ersten Sumpfstrom enthaltene Tetramethylglycolid kristallisiert. Schritt b) des erfindungsgemäßen Verfahrens lautet dann:
b) Kristallisation des in dem ersten Sumpfstrom erhaltenen Tetramethylglycolids unter Erhalt von kristallisiertem Tetramethylglycolid und einer Produktflüssigphase.

Schritt a1) wird vorzugsweise in einer zweiten Kolonne durchgeführt. Die zweite Kolonne ist bevorzugt von der vom ersten Reaktor bevorzugt umfassten Kolonne in Schritt a) verschieden. In diese zweite Kolonne wird vorzugsweise kontinuierlich Produktmischung zugeführt und der erste Sumpfstrom und der erste Kopfstrom werden kontinuierlich entnommen.

Die Destillation in Schritt a1) wird bevorzugt bei einer zweiten Temperatur durchgeführt. Die zweite Temperatur bezieht sich auf die Sumpftemperatur während der Destillation und liegt vorzugsweise im Bereich von 150 °C bis 200 °C, besonders bevorzugt im Bereich von 160 °C bis 190 °C.

Bei der Destillation in Schritt a1) wird üblicherweise TMG teilweise gespalten, wobei sich Aceton, Methacrylsäure und Kohlenmonooxid bilden. Daher enthält der bei der Destillation erhaltene erste Kopfstrom üblicherweise zusätzlich Methacrylsäure und/oder Aceton.

Enthält die Produktmischung zusätzlich Methacrylsäure und/oder Aceton, so enthält der bei der Destillation erhaltene erste Kopfstrom üblicherweise ebenfalls zusätzlich Methacrylsäure und/oder Aceton.

Der erste Kopfstrom kann zumindest teilweise in die Reaktionsmischung rückgeführt werden. Bevorzugt wird der in Schritt a1) erhaltene erste Kopfstrom in Schritt a) rückgeführt.

Bevorzugt ist daher auch ein Verfahren, bei dem der in Schritt a1) erhaltene erste Kopfstrom in Schritt a) rückgeführt wird.

In Schritt c) wird polymerisierbares Tetramethylglycolid erhalten.

Gegenstand der vorliegenden Erfindung ist daher auch polymerisierbares Tetramethylglycolid erhältlich nach dem erfindungsgemäßen Verfahren.

Üblicherweise enthält das im erfindungsgemäßen Verfahren erhaltene polymerisierbare Tetramethylglycolid zusätzlich Nebenprodukte.

Vorzugsweise umfassen die Nebenprodukte
10 Gew.-ppm bis 1000 Gew.-ppm 2-Hydroxyisobuttersäure,
10 Gew.-ppm bis 1000 Gew.-ppm Wasser und
10 Gew.-ppm bis 5000 Gew.-ppm Di-2-Hydroxyisobuttersäure, jeweils bezogen auf das Gesamtgewicht des polymerisierbaren Tetramethylglycolids.

Enthält die Produktmischung und/oder der erste Sumpfstrom, aus der das polymerisierbare Tetramethylglycolid in Schritt b) kristallisiert wird, zusätzlich Methacrylsäure, so umfassen die Nebenprodukte, die das polymerisierbare Tetramethylglycolid enthält, üblicherweise ebenfalls Methacrylsäure.

Beispielsweise umfassen die Nebenprodukte dann zusätzlich 1 Gew.-ppm bis 1000 Gew.-ppm Methacrylsäure, bezogen auf das Gesamtgewicht des polymerisierbaren Tetramethylglycolids.

Bevorzugt ist daher auch polymerisierbares Tetramethylglycolid, wobei die Nebenprodukte zusätzlich 1 Gew.-ppm bis 1000 Gew.-ppm Methacrylsäure umfassen, bezogen auf das Gesamtgewicht des polymerisierbaren Tetramethylglycolids.

Enthält die Produktmischung und/oder der erste Sumpfstrom, aus der das polymerisierbare Tetramethylglycolid in Schritt b) kristallisiert wird, zusätzlich oligomere 2-Hydroxyisobuttersäure, so umfassen die Nebenprodukte, die das polymerisierbare Tetramethylglycolid enthält, üblicherweise ebenfalls oligomere 2-Hydroxyisobuttersäure.

Beispielsweise umfassen die Nebenprodukte zusätzlich 1 Gew.-ppm bis 5000 gew.-ppm oligomere 2-Hydroxyisobuttersäure, bezogen auf das Gesamtgewicht des polymerisierbaren Tetramethylglycolids.

Bevorzugt ist daher auch polymerisierbares Tetramethylglycolid, wobei die Nebenprodukte zusätzlich 1 Gew.-ppm bis 5000 Gew.-ppm oligomere 2-Hydroxyisobuttersäure umfassen, bezogen auf das Gesamtgewicht des polymerisierbaren Tetramethylglycolids.

Enthält die Produktmischung und/oder der erste Sumpfstrom, aus der das polymerisierbare Tetramethylglycolid in Schritt b) kristallisiert wird, zusätzlich mindestens ein Diketon von Morpholin, das mit vier Methylgruppen substituiert ist, so umfassen die Nebenprodukte, die das polymerisierbare Tetramethylglycolid enthält, üblicherweise ebenfalls zusätzlich mindestens ein Diketon von Morpholin, das mit vier Methylgruppen substituiert ist.

Beispielsweise enthalten die Nebenprodukte zusätzlich 1 Gew.-ppm bis 500 Gew.-ppm mindestens eines Diketons von Morpholin, das mit vier Methylgruppen substituiert ist, bezogen auf das Gesamtgewicht des polymerisierbaren Tetramethylglycolids.

Bevorzugt ist daher auch polymerisierbares Tetramethylglycolid, wobei die Nebenprodukte zusätzlich 1 Gew.-ppm bis 500 Gew.-ppm mindestens eines Diketons von Morpholin, das mit vier Methylgruppen substituiert ist, umfassen, bezogen auf das Gesamtgewicht des polymerisierbaren Tetramethylglycolids.

Es versteht sich von selbst, dass sich die Gew.-ppm der Nebenprodukte auf das Gesamtgewicht des polymerisierbaren Tetramethylglycolids inklusive der zusätzlich enthaltenen Nebenprodukte bezieht.

Das Verhältnis der molaren Summen aller aziden Protonen der Nebenprodukte zu der molaren Menge des reinen polymerisierbaren Tetramethylglycolids liegt vorzugsweise im Bereich von 0,05 bis 0,0001.

Bevorzugt ist daher auch polymerisierbares Tetramethylglycolid, wobei das polymerisierbare Tetramethylglycolid zusätzlich Nebenprodukte enthält, wobei die Nebenprodukte
10 Gew.-ppm bis 1000 Gew.-ppm 2-Hydroxyisobuttersäure,
10 Gew.-ppm bis 1000 Gew.-ppm Wasser und
10 Gew.-ppm bis 5000 Gew.-ppm Di-2-Hydroxyisobuttersäure umfassen, bezogen auf das Gesamtgewicht des polymerisierbaren Tetramethylglycolids und wobei
das Verhältnis der molaren Summen aller aziden Protonen der Nebenprodukte zur molaren Menge des polymerisierbaren Tetramethylglycolids im Bereich von 0,05 bis 0,0001 liegt.

Unter aziden Protonen der Nebenprodukte werden alle OH-, NH-, PH- und SH-aziden Protonen der Nebenprodukte, bevorzugt alle OH- und NH-aziden Protonen der Nebenprodukte verstanden.

In einer alternativen Ausführungsform des erfindungsgemäßen Verfahrens werden zusätzlich oder alternativ, vorzugsweise alternativ, zu den Schritten b) und c) die folgenden Schritte b1) und b2) durchgeführt:
b1) Destillation der in Schritt a) erhaltenen Produktmischung unter Erhalt eines Tetramethylglycolid-haltigen Destillats,
b2) Waschen des in Schritt b1) erhaltenen Tetramethylglycolid-haltigen Destillats unter Erhalt des polymerisierbaren Tetramethylglycolids.

Die Destillation in Schritt b1) kann nach dem Fachmann bekannten Methoden erfolgen. Vorzugsweise erfolgt sie zumindest zweitstufig. Üblicherweise werden in diesem Fall zunächst in einem ersten Destillationsschritt Komponenten mit einer niedrigeren Siedetemperatur als Tetramethylglycolid aus der Produktmischung abgetrennt. In einem zweiten Destillationsschritt wird dann Tetramethylglycolid als Kopfstrom abgetrennt unter Erhalt des Tetramethylglycolid-haltigen Destillats.

Das Tetramethylglycolid-haltige Destillat enthält Tetramethylglycolid. Darüber hinaus enthält es üblicherweise noch bei der Destillation aus Tetramethylglycolid gebildete Nebenprodukte wie beispielsweise Aceton und/oder Methacrylsäure. Darüber hinaus kann es beispielsweise Reste der in der Produktmischung enthaltenen Komponenten enthalten, insbesondere mindestens ein Diketon von Morpholin, das mit vier Methylgruppen substituiert ist.

In Schritt b2) wird daher das Tetramethylglycolid-haltige Destillat gewaschen. Das Waschen kann nach dem Fachmann bekannten Methoden erfolgen, beispielsweise mit Wasser, vorzugweise mit basischem Wasser. Dadurch können Nebenprodukte abgetrennt werden unter Erhalt von polymerisierbarem Tetramethylglycolid. Es ist möglich, dass in Schritt b2) das Tetramethylglycolid-haltige Destillat zunächst wie vorstehend beschrieben gewaschen wird und anschließend durch Adsorption und/oder Absorption aufgereinigt wird unter Erhalt des polymerisierbaren Tetramethylglycolids. Die Adsorption und/oder Absorption kann beispielsweise an Silica, Aluminiumoxid und/oder einem Ionentauscher erfolgen. Diese Verfahren sind dem Fachmann als solche bekannt. Die Adsorption und/oder Absorption ist insbesondere vorteilhaft zur Entfernung des gegebenenfalls in dem Tetramethylglycolid-haltigen Destillat enthaltenen mindestens einem Diketon von Morpholin, das mit vier Methylgruppen substituiert ist.

### Beispiele

### Polymerisation verschiedener TMG-Chargen

Die hier durchgeführte Polymerisation ist zum Testen der hergestellten TMG-Chargen geeignet. Sie bestimmt, ob mit einem TMG die Polymerisation grundsätzlich durchführbar ist.

Die zu polymerisierende TMG-Menge (typischerweise 30 g) wurde zunächst in einen Erlenmeyerkolben mit Magnetrührfisch eingewogen. Der Erlenmeyerkolben wurde dann in einem Vakuumtrockenschrank bei 0 mbar für eine Stunde auf 130 °C temperiert. Dabei schmolz das TMG auf und Restfeuchtigkeit aus dem TMG bzw. der Glasoberfläche des Kolbens wurden entfernt. Nach einer Stunde wurde der Trockenschrank belüftet, der Kolben mit einem Gummistopfen verschlossen und in ein auf 130°C vortemperiertes Ölbad eingehängt und per Magnetrührer durchmischt. Anschließend wurde eine Kanüle durch den Stopfen gestochen und der Kolben mit Argon gespült. Dann wurde. mit einer Spritze über die Kanüle (für die gewünschte Molmasse erforderliche Menge) 0,15mol% Lithium tert Butylat (als t-BuOLi gelöst in THF, Reinstoff t-BuOLi bezogen auf Einwaage TMG) Katalysator zugegeben. War das Material zur Polymerisation geeignet, wurde nach ca. 5 bis 15 min eine Viskosität erreicht, bei der der Magnetrührfisch stehen blieb. Anschließend wurde die Polymerisation noch 6-8h fortgesetzt.

Die Reinheiten der TMG-Chargen, welche analog zu Beispiel 17 und einem Umsatzgrad an 2-Hydroxyisobuttersäure kleiner 95% hergestellt wurden, sowie deren Polymerisationsfähigkeit ist in Tabelle 1 angegeben.

**Tabelle 1**

| **Beispiel** | **TMG [Gew.-%]** | **H₂O [Gew.-%]** | **2-HIBS [Gew.-%]** | **Di-2-HIBS [Gew.-%]** | **MAS [Gew.-%]** | **Erfolgreiche Polymerisation** |
|---|---|---|---|---|---|---|
| **V1 (Rohprodukt)** | 98,9 | 0,003 | 0,1 | 0,989 | 0,005 | Nein |
| **B2 (Reinprodukt)** | 99,5 | 0,004 | 0,01 | 0,313 | 0,003 | ja |
| **V3 (Rohprodukt)** | 92,1 | 0,012 | 5,4 | 2,4 | 0,009 | nein |
| **V4 (destilliert)** | 90,4 | 0,098 | 0,36 | 1,3 | 0,002 | nein |
| **V5 (Schmelzkristallisation)** | 99,2 | 0,023 | 0,3 | 0,41 | 0,004 | nein |
| **B6 (Schmelzkristallisation)** | 99,5 | 0,018 | 0,08 | 0,32 | 0,003 | ja |
| **B7 (Schmelzkristallisation)** | 99,7 | 0,012 | 0,05 | 0,22 | 0,002 | ja |
| **V8 (kristallisiert i-PrOH + gewaschen)** | 97,1 | 0,085 | 0,07 | 0,81 | 0,006 | nein |
| **V9 (kristallisiert i-PrOH + gewaschen + getrocknet)** | 99,1 | 0,009 | 0,08 | 0,71 | 0,002 | nein |
| **V10 (2x kristallisiert i-PrOH + gewaschen** | 98,3 | 0,031 | 0,039 | 0,19 | 0,006 | nein |
| **B11 (2x kristallisiert i-PrOH + gewaschen + getrocknet)** | 99,71 | 0,0089 | 0,078 | 0,18 | 0,003 | ja |
| **V12 (2x kristallisiert i-PrOH + gewaschen** + **getrocknet + offen gelagert)** | 99,59 | 0,12 | 0,078 | 0,18 | 0,003 | nein |
| **V13 (kristallisiert Toluol)** | 90,2 | 0,07 | 0,015 | 0,7 | 0,053 | nein |
| **B14 (2x kristallisiert aus Toluol)** | 90,5 | 0,03 | 0,01 | 0,25 | 0,027 | ja |
| **B15 (kristallisiert Pentan)** | 98 | 0,062 | 0,089 | 0,4 | 0,025 | ja |
| **B16 (extrahiert Toluol, kristallisiert Pentan)** | 96,4 | 0,034 | 0,068 | 0,3 | 0,015 | ja |

### Beispiel B17: Herstellung von TMG aus 2-HIBS:

In einem gerührten 5L-Doppelmantelglasbehälter (erster Reaktor) wurden 3112,6 g Reaktionsmischung der folgenden Zusammensetzung vorgelegt (die zu 100 Gew.-% fehlenden Mengen ergeben sich einerseits aus der Messgenauigkeit der verwendeten HPLC und/oder GC, andererseits handelt es sich hierbei um nicht detektierte Nebenkomponenten):

| | |
|---|---|
| 2-HIBS | 79,3 Gew.-% |
| 2-DiHIBS | 16,2 Gew.-% |
| TMG | 1,64 Gew.-% |

Der Behälter war mit einer Kolonne ausgestattet, die unten mit drei SULZER EX-Packungen aus Hastelloy, darüber mit Glas-Raschigringen gefüllt war. Der Behälter wurde mit Thermalöl beheizt. Behälter und Kolonne wurden auf 300 mbara evakuiert und auf eine Innentemperatur von 165 bis 170 °C aufgeheizt. Über einen Zeitraum von 9 h (Stunden) wurde Wasser über Kopf abgezogen und sowohl erster Reaktor als auch Destillat nach 0 Stunden (h), 1 h, 2 h, 4 h, 6 h, 8 h und 9 h beprobt. Nach 9 h enthält der Reaktor 2619 g Produktmischung (im Weiteren als Roh-TMG bezeichnet), im Destillat wurden 442,7 g gesammelt und nach Abschluss des Versuches wurden einmalig 17,8 g aus der vorgeschalteten Kältefalle des Vakuum entnommen. Die Zusammensetzung der Proben Reaktionsmischung und während der Umsetzung im ersten Reaktor ist in Tabelle 2 gezeigt (die zu 100 Gew.-% fehlenden Mengen ergeben sich aus der Messgenauigkeit der verwendeten Analysemethoden; KF: Karl-Fischer Titration).

**Tabelle 2**

| | | | **Reaktionsmischung** | **erster Reaktor** | | | | | |
|---|---|---|---|---|---|---|---|---|---|
| **Versuchsdauer / h** | | | 0,0 | 1,0 | 2,0 | 4,0 | 6,0 | 8,0 | 9,0 |
| **Umsatzgrad 2-HiBS / %** | | | | 15,5 | 31,4 | 52,6 | 65,0 | 73,7 | 78,2 |
| **Analyti k** | | | | | | | | | |
| **HPLC** | 2-HIBS | Gew.-% | 79,3 | 69,4 | 58,1 | 41,8 | 31,8 | 24,3 | 20,4 |
| **HPLC** | MAS | Gew.-% | 0,0 | 0,1 | 0,1 | 0,1 | 0,1 | 0,0 | 0,040 |
| **HPLC** | Di-2-HIBS | Gew.-% | 16,2 | 8,5 | 9,7 | 7,9 | 6,8 | 5,2 | 4,7 |
| **HPLC** | TMG | Gew.-% | 1,64 | 18,6 | 28,5 | 46,7 | 58,8 | 67,6 | 71,6 |
| **GC** | Aceton | Gew.-% | | | | | | | |
| **KF** | H₂O | Gew.-% | | 0,9 | | 0,64 | | | 0,6 |
| **Massen** | | | | | | | | | |
| | m Gesamt | g | 3112,60 | 3006 | 2913 | 2799 | 2719 | 2668 | 2619 |

Die Zusammensetzung des Destillats und der Kältefalle zum Abschluss des Versuch ist in Tabelle 3 gezeigt (die zu 100 Gew.-% fehlenden Mengen ergeben sich aus der Messgenauigkeit der verwendeten Analysemethoden; KF: Karl-Fischer Titration).

**Tabelle 3**

| | | | **Destillat** | | | | | | **Kältefalle** |
|---|---|---|---|---|---|---|---|---|---|
| **Versuchsdauer / h** | | | 1,0 | 2,0 | 4,0 | 6,0 | 8,0 | 9,0 | 9,0 |
| **Analytik** | | | | | | | | | |
| **HPLC** | 2-HIBS | Gew.-% | 0,0 | 0,0 | 0,0 | 0,0 | 0,0 | 0,0 | 0,0 |
| **HPLC** | MAS | Gew.-% | 3,1 | 8,7 | 12,1 | 12,3 | 13,3 | 10,8 | 4,7 |
| **HPLC** | Di-2-HIBS | Gew.-% | 0,0 | 0,0 | 0,0 | 0,0 | 0,0 | 0,0 | 0,0 |
| **HPLC** | TMG | Gew.-% | 0,0 | 0,0 | 0,0 | 0,0 | 0,0 | 0,0 | 0,0 |
| **GC** | Aceton | Gew.-% | 0,1 | 0,2 | 0,1 | 0,1 | 0,1 | 0,2 | 44,4 |
| **KF** | H₂O | Gew.-% | 86,8 | 82,1 | 83,7 | 82,4 | 82,3 | 78,6 | 45,1 |
| **Massen** | | | | | | | | | |
| | m Gesamt | g | 102,2 | 89,5 | 109,6 | 71,4 | 48,9 | 21,1 | 17,8 |

Obwohl der Umsatz der Reaktion kontinuierlich ansteigt, fängt die Selektivität (anhand des Destillats, das statt >95% nur noch 89% Wasser und MAS enthält) nach 8h oder etwa 75% Umsatz (basierend auf 2-HIBS) an zufallen. Es ist daher zweckmäßig die Reaktion nicht in den Vollumsatz zu treiben und vorzeitig zu beenden.

Die folgenden Vergleichsbeispiele V18, V19 und V20 geben eine allgemeine Übersicht über die Kristallisation von TMG aus der Mutterlauge (Vergleichsbeispiel 18), die Destillation von TMG (Vergleichsbeispiel 19) und die Extraktion von TMG (Vergleichsbeispiel 20) wie sie beispielsweise auch vorstehend für die verschiedenen TMG-Chargen durchgeführt wurden. Die in den nachfolgenden Vergleichsbeispielen zu 100 Gew.-% fehlenden Mengen ergeben sich aus der Messgenauigkeit der verwendeten Analysemethoden.

### Vergleichsbeispiel V18 Kristallisation von TMG aus der Mutterlauge:

Nach Ende einer Synthese ausgehend von Beispiel B17 wurde das Vakuum aufgehoben und das Roh-TMG über Nacht auf ca. 58°C abgekühlt. Dabei kristallisierte reines TMG aus, eine Produktflüssigphase aus TMG, 2-HIBS, 2-DiHIBS und MAS blieb zurück. Diese Produktflüssigphase kann wieder der Umsetzung zugeführt werden. Der Behälterinhalt wird dann in zwei Fraktionen mit einer Vakuumpumpe durch einen auf ca. 58°C beheizten Filter gesaugt. Der Filterkuchen hatte die folgende Zusammensetzung:

| | |
|---|---|
| 2-HIBS | 5,4 Gew.-% |
| MAS | 0,009 Gew.-% |
| Di-2-HIBS | 2,4 Gew.-% |
| TMG | 92,1 Gew.-% |

Das abgesaugte Filtrat (Produktflüssigphase) hatte die folgende Zusammensetzung:

| | |
|---|---|
| 2-HIBS | 30,3 Gew.-% |
| MAS | 0,1 Gew.-% |
| Di-2-HIBS | 9,9 Gew.-% |
| TMG | 55,1 Gew.-% |

Der Filterkuchen wurde dann mit 300 g 2-Propanol gewaschen: Die Zusammensetzung nach dem Waschen betrug:

| | |
|---|---|
| 2-HIBS | 0,07 Gew.-% |
| MAS | 0,006 Gew.-% |
| Di-2-HIBS | 0,81 Gew.-% |
| TMG | 97,1 Gew.-% |

Die Kristalle wurden dann für 48h bei Raumtemperatur unter Vakuum (< 50 mbar) bis zur Massenkonstanz getrocknet. Nach dem Trocknen wurden insgesamt 517 g TMG der folgenden Zusammensetzung erhalten:

| | |
|---|---|
| 2-HIBS | 0,08 Gew.-% |
| MAS | 0,002 Gew.-% |
| Di-2-HIBS | 0,71 Gew.-% |
| TMG | 99,1 Gew.-% |

### Vergleichsbeispiel V19 Destillation von TMG

Die destillative Aufreinigung eines Roh-TMG ausgehend von Beispiel B17 wurde an einer DN50 Kolonnen von 2m Länge durchgeführt, gefüllt mit SULZER EX-Packungen aus Hastelloy. Der Kolonnensumpf wurde beheizt auf eine Innentemperatur von 189°C, die Kolonne wurde begleitbeheizt auf ca. 183 bis 185°C. Die Destillation wurde mit einem Rücklaufverhältnis von 3:1 betrieben. Die Zusammensetzung der Ein- und Ausgangsströme betrug:
Feed: 480 g/h

| | |
|---|---|
| 2-HIBS | 13,4 Gew.-% |
| MAS | 0,06 Gew.-% |
| Di-2-HIBS | 6,2 Gew.-% |
| TMG | 77,2 Gew.-% |

Sumpf: 195 g/h

| | |
|---|---|
| 2-HIBS | 0,36 Gew.-% |
| MAS | 0 Gew.-% |
| Di-2-HIBS | 1,3 Gew.-% |
| TMG | 90,4 Gew.-% |

Rest (Oligomere)
Kopf: 285 g/h

| | |
|---|---|
| 2-HIBS | 25,8 Gew.-% |
| MAS | 0,12 Gew.-% |
| Di-2-HIBS | 1,0 Gew.-% |
| TMG | 73 Gew.-% |

Das Kopfprodukt kann wieder der Synthese zugeführt werden.

### Vergleichsbeispiel V20 Extraktion von TMG

RohTMG aus einem vorherigen Beispiel mit folgender Zusammensetzung wurden zunächst im Massenverhältnis 1 : 1,75 in Toluol gelöst:

| | |
|---|---|
| 2-HIBS | 0,4 Gew.-% |
| Di-2-HIBS | 7,4 Gew.-% |
| TMG | 92,4 Gew.-% |

Etwaige Anteile an nicht löslichem Feststoff wurden abfiltriert. Die toluolische Lösung wurde anschließend im Rotationsverdampfer getrocknet. Die erhaltenen Kristalle wurden separiert und ein Teil nochmal mit wenig Petrolether gewaschen um verbliebenes Toluol zu entfernen. Dann wurden die Kristalle im Vakuumtrockenschrank bei 50°C und 0 mbar bis zur Massenkonstanz getrocknet.

## Patentansprüche

1. Verfahren zur Herstellung von polymerisierbarem Tetramethylglycolid umfassend die folgenden Schritte a) bis c):
a) Umsetzen einer Reaktionsmischung, die 2-Hydroxyisobuttersäure enthält, in einem ersten Reaktor bei einer ersten Temperatur, die im Bereich von 150 °C bis 200 °C liegt, und einem ersten Druck, der im Bereich von 100 mbar bis 1013 mbar liegt, wobei der Umsatzgrad der 2-Hydroxyisobuttersäure bei der Umsetzung bei maximal 95 % liegt, unter Erhalt einer Produktmischung, die
5 bis 50 Gew.-% 2-Hydroxyisobuttersäure,
25 bis 85 Gew.-% Tetramethylglycolid und
5 bis 25 Gew.-% Di-2-Hydroxyisobuttersäure enthält, jeweils bezogen **auf** das Gesamtgewicht der Produktmischung,
b) Kristallisation des in der Produktmischung enthaltenen Tetramethylglycolids unter Erhalt von kristallisiertem Tetramethylglycolid und einer Produktflüssigphase,
c) Abtrennung des in Schritt b) kristallisierten Tetramethylglycolids von der Produktflüssigphase unter Erhalt des polymerisierbaren Tetramethylglycolids.

2. Verfahren gemäß Anspruch 1, **dadurch gekennzeichnet, dass** die Reaktionsdauer der Umsetzung in Schritt a) im Bereich von 1 Stunde bis 24 Stunden liegt.

3. Verfahren gemäß Anspruch 1 oder 2, **dadurch gekennzeichnet, dass** der erste Reaktor mindestens eine Kolonne umfasst.

4. Verfahren gemäß einem der Ansprüche 1 bis 3, **dadurch gekennzeichnet, dass** im Anschluss an Schritt a) und vor Schritt b) der folgende Schritt a1) durchgeführt wird
a1) Destillation der in Schritt a) erhaltenen Produktmischung bei einem zweiten Druck, der im
Bereich von 100 mbar bis 500 mbar liegt, unter Erhalt eines ersten Sumpfstroms und
eines ersten Kopfstroms, wobei der erste Sumpfstrom
mindestens 80 Gew.-% Tetramethylglycolid,
höchstens 1 Gew.-% 2-Hydroxyisobuttersäure,
höchstens 2 Gew.-% Di-2-Hydroxyisobuttersäure und
mindestens 5 Gew.-% oligomere 2-Hydroxyisobuttersäure enthält, jeweils bezogen **auf** das Gesamtgewicht des ersten Sumpfstroms,
und wobei der erste Kopfstrom
0,1 bis 5 Gew.-% Di-2-Hydroxyisobuttersäure,
10 bis 50 Gew.-% 2-Hydroxyisobuttersäure und
40 bis 85 Gew.-% Tetramethylglycolid enthält, jeweils bezogen **auf** das Gesamtgewicht des ersten Kopfstroms,
und in Schritt b) dann das in dem ersten Sumpfstrom enthaltene Tetramethylglycolid kristallisiert wird.

5. Verfahren gemäß einem der Ansprüche 1 bis 4, **dadurch gekennzeichnet, dass** die Umsetzung in Schritt a) autokatalytisch abläuft.

6. Verfahren gemäß einem der Ansprüche 1 bis 5, **dadurch gekennzeichnet, dass** die Reaktionsmischung zusätzlich mindestens eine weitere Komponente ausgewählt aus der Gruppe bestehend aus Tetramethylglycolid, Di-2-Hydroxyisobuttersäure und oligomere 2-Hydroxyisobuttersäure enthält.

7. Verfahren gemäß einem der Ansprüche 1 bis 6, **dadurch gekennzeichnet, dass** die Kristallisation in Schritt b) bei einer zweiten Temperatur im Bereich von 50 °C bis 80 °C stattfindet.

8. Verfahren gemäß einem der Ansprüche 1 bis 7, **dadurch gekennzeichnet, dass** die in Schritt c) erhaltene Produktflüssigphase in Schritt a) rückgeführt wird.

9. Verfahren gemäß einem der Ansprüche 1 bis 8, **dadurch gekennzeichnet, dass** die in Schritt a) erhaltene Produktmischung zusätzlich oligomere 2-Hydroxyisobuttersäure enthält und diese in Gegenwart eines Zinn(IV)oxidkatalysators gespalten und in Schritt a) rückgeführt wird.

10. Verfahren gemäß einem der Ansprüche 1 bis 9, **dadurch gekennzeichnet, dass** die in Schritt a) umgesetzte Reaktionsmischung erhältlich ist durch ein Verfahren zur Herstellung von Acetoncyanhydrin durch Umsetzung von Aceton mit Blausäure.

11. Polymerisierbares Tetramethylglycolid erhältlich nach einem Verfahren gemäß einem der Ansprüche 1 bis 10.

12. Polymerisierbares Tetramethylglycolid gemäß Anspruch 11, **dadurch gekennzeichnet, dass** das polymerisierbare Tetramethylglycolid zusätzlich Nebenprodukte enthält, wobei die Nebenprodukte
10 Gew.-ppm bis 1000 Gew.-ppm 2-Hydroxyisobuttersäure,
10 Gew.-ppm bis 1000 Gew.-ppm Wasser und
10 Gew.-ppm bis 5000 Gew.-ppm Di-2-Hydroxyisobuttersäure umfassen, bezogen auf das Gesamtgewicht des polymerisierbaren Tetramethylglycolids und wobei
das Verhältnis der molaren Summen aller aziden Protonen der Nebenprodukte zur molaren Menge des polymerisierbaren Tetramethylglycolids im Bereich von 0,05 bis 0,0001 liegt.

13. Polymerisierbares Tetramethylglycolid gemäß Anspruch 12, **dadurch gekennzeichnet, dass** die Nebenprodukte zusätzlich 1 Gew.-ppm bis 1000 Gew.-ppm Methacrylsäure umfassen, bezogen auf das Gesamtgewicht des polymerisierbaren Tetramethylglycolids.

14. Polymerisierbares Tetramethylglycolid gemäß Anspruch 12 oder 13, **dadurch gekennzeichnet, dass** die Nebenprodukte zusätzlich 1 Gew.-ppm bis 5000 Gew.-ppm oligomere 2-Hydroxyisobuttersäure umfassen, bezogen auf das Gesamtgewicht des polymerisierbaren Tetramethylglycolids.

15. Polymerisierbares Tetramethylglycolid gemäß einem der Ansprüche 12 bis 14, **dadurch gekennzeichnet, dass** die Nebenprodukte zusätzlich 1 Gew.-ppm bis 500 Gew.-ppm mindestens eines Diketons von Morpholin, das mit vier Methylgruppen substituiert ist, umfassen, bezogen auf das Gesamtgewicht des polymerisierbaren Tetramethylglycolids.

## Claims

1. Process for preparing polymerizable tetramethylglycolide comprising the following steps a) to c):
a) reaction of a reaction mixture containing 2-hydroxyisobutyric acid in a first reactor at a first temperature in the range from 150°C to 200°C and a first pressure in the range from 100 mbar to 1013 mbar, where the degree of conversion of the 2-hydroxyisobutyric acid in the reaction is at most 95%, to obtain a product mixture containing
5% to 50% by weight of 2-hydroxyisobutyric acid,
25% to 85% by weight of tetramethylglycolide and
5% to 25% by weight of di-2-hydroxyisobutyric acid, in each case based on the total weight of the product mixture,
b) crystallization of the tetramethylglycolide present in the product mixture to obtain crystallized tetramethylglycolide and a product liquid phase,
c) separation of the tetramethylglycolide crystallized in step b) from the product liquid phase to obtain the polymerizable tetramethylglycolide.

2. Process according to Claim 1, **characterized in that** the reaction duration of the reaction in step a) is in the range from 1 hour to 24 hours.

3. Process according to Claim 1 or 2, **characterized in that** the first reactor comprises at least one column.

4. Process according to any of Claims 1 to 3, **characterized in that** after step a) and before step b) the following step a1) is carried out
a1) distillation of the product mixture obtained in step a) at a second pressure in the range from 100 mbar to 500 mbar, to obtain a first bottom stream and a first overhead stream, where the first bottom stream contains at least 80% by weight of tetramethylglycolide,
at most 1% by weight of 2-hydroxyisobutyric acid,
at most 2% by weight of di-2-hydroxyisobutyric acid and at least 5% by weight of oligomeric 2-hydroxyisobutyric acid, in each case based on the total weight of the first bottom stream,
and where the first overhead stream contains
0.1% to 5% by weight of di-2-hydroxyisobutyric acid,
10% to 50% by weight of 2-hydroxyisobutyric acid and
40% to 85% by weight of tetramethylglycolide, in each case based on the total weight of the first overhead stream,
and in step b) the tetramethylglycolide present in the first bottom stream is then crystallized.

5. Process according to any of Claims 1 to 4, **characterized in that** the reaction in step a) proceeds autocatalytically.

6. Process according to any of Claims 1 to 5, **characterized in that** the reaction mixture additionally contains at least one further component selected from the group consisting of tetramethylglycolide, di-2-hydroxyisobutyric acid and oligomeric 2-hydroxyisobutyric acid.

7. Process according to any of Claims 1 to 6, **characterized in that** the crystallization in step b) takes place at a second temperature in the range from 50°C to 80°C.

8. Process according to any of Claims 1 to 7, **characterized in that** the product liquid phase obtained in step c) is recycled into step a).

9. Process according to any of Claims 1 to 8, **characterized in that** the product mixture obtained in step a) additionally contains oligomeric 2-hydroxyisobutyric acid and this is cleaved in the presence of a tin (IV) oxide catalyst and recycled into step a).

10. Process according to any of Claims 1 to 9, **characterized in that** the reaction mixture reacted in step a) is obtainable by a process for preparing acetone cyanohydrin by reacting acetone with hydrocyanic acid.

11. Polymerizable tetramethylglycolide obtainable by a process according to any of Claims 1 to 10.

12. Polymerizable tetramethylglycolide according to Claim 11, **characterized in that** the polymerizable tetramethylglycolide additionally contains byproducts, where the byproducts comprise
10 ppm by weight to 1000 ppm by weight of 2-hydroxyisobutyric acid,
10 ppm by weight to 1000 ppm by weight of water and
10 ppm by weight to 5000 ppm by weight of di-2-hydroxyisobutyric acid, based on the total weight of the polymerizable tetramethylglycolide, and where
the ratio of the molar sum totals of all acidic protons of the byproducts to the molar amount of the polymerizable tetramethylglycolide is in the range from 0.05 to 0.0001.

13. Polymerizable tetramethylglycolide according to Claim 12, **characterized in that** the byproducts additionally comprise 1 ppm by weight to 1000 ppm by weight of methacrylic acid, based on the total weight of the polymerizable tetramethylglycolide.

14. Polymerizable tetramethylglycolide according to Claim 12 or 13, **characterized in that** the byproducts additionally comprise 1 ppm by weight to 5000 ppm by weight of oligomeric 2-hydroxyisobutyric acid, based on the total weight of the polymerizable tetramethylglycolide.

15. Polymerizable tetramethylglycolide according to any of Claims 12 to 14, **characterized in that** the byproducts additionally comprise 1 ppm by weight to 500 ppm by weight of at least one diketone of morpholine that is substituted by four methyl groups, based on the total weight of the polymerizable tetramethylglycolide.

## Revendications

1. Procédé pour la préparation de tétraméthylglycolide polymérisable, comprenant les étapes a) à c) suivantes :
a) transformation d'un mélange réactionnel qui contient de l'acide 2-hydroxyisobutyrique dans un premier réacteur à une première température, qui se situe dans la plage de 150°C à 200°C, et à une première pression, qui se situe dans la plage de 100 mbars à 1013 mbars, le degré de conversion de l'acide 2-hydroxyisobutyrique lors de la transformation se situant à au maximum 95%, avec obtention d'un mélange de produits, qui contient
5 à 50% en poids d'acide 2-hydroxyisobutyrique,
25 à 85% en poids de tétraméthylglycolide et
5 à 25% en poids de diacide 2-hydroxyisobutyrique, à chaque fois par rapport au poids total du mélange de produits,
b) cristallisation du tétraméthylglycolide contenu dans le mélange de produits avec obtention de tétraméthylglycolide cristallisé et d'une phase liquide de produits,
c) séparation du tétraméthylglycolide cristallisé dans l'étape b) de la phase liquide de produits avec obtention du tétraméthylglycolide polymérisable.

2. Procédé selon la revendication 1, **caractérisé en ce que** la durée de réaction de la transformation dans l'étape a) se situe dans la plage de 1 heure à 24 heures.

3. Procédé selon la revendication 1 ou 2, **caractérisé en ce que** le premier réacteur comprend au moins une colonne.

4. Procédé selon l'une des revendications 1 à 3, **caractérisé en ce que** l'étape a1) suivante est effectuée après l'étape a) et avant l'étape b)
a1) distillation du mélange de produits obtenu dans l'étape a) à une deuxième pression, qui se situe dans la plage de 100 mbars à 500 mbars, avec obtention d'un premier flux de fond et d'un premier flux de tête, le premier flux de fond contenant
au moins 80% en poids de tétraméthylglycolide,
au plus 1% en poids d'acide 2-hydroxyisobutyrique,
au plus 2% en poids de diacide 2-hydroxyisobutyrique et au moins 5% en poids d'acide 2-hydroxyisobutyrique oligomère, à chaque fois par rapport au poids total du premier flux de fond,
et le premier flux de tête contenant
0,1 à 5% en poids de diacide 2-hydroxyisobutyrique,
10 à 50% en poids d'acide 2-hydroxyisobutyrique et
40 à 85% en poids de tétraméthylglycolide , à chaque fois par rapport au poids total du premier flux de tête,
et le tétraméthylglycolide contenu dans le premier flux de fond est ensuite cristallisé dans l'étape b).

5. Procédé selon l'une des revendications 1 à 4, **caractérisé en ce que** la transformation dans l'étape a) se déroule de manière autocatalytique.

6. Procédé selon l'une des revendications 1 à 5, **caractérisé en ce que** le mélange réactionnel contient en plus au moins un autre composant choisi dans le groupe constitué par le tétraméthylglycolide, le diacide 2-hydroxyisobutyrique et l'acide 2-hydroxyisobutyrique oligomère.

7. Procédé selon l'une des revendications 1 à 6, **caractérisé en ce que** la cristallisation dans l'étape b) a lieu à une deuxième température dans la plage de 50°C à 80°C.

8. Procédé selon l'une des revendications 1 à 7, **caractérisé en ce que** la phase liquide de produits obtenue dans l'étape c) est recyclée dans l'étape a).

9. Procédé selon l'une des revendications 1 à 8, **caractérisé en ce que** le mélange de produits obtenu dans l'étape a) contient en plus de l'acide 2-hydroxyisobutyrique oligomère et celui-ci est dissocié en présence d'un catalyseur de type oxyde d'étain (IV) et recyclé dans l'étape a).

10. Procédé selon l'une des revendications 1 à 9, **caractérisé en ce que** le mélange réactionnel transformé dans l'étape a) peut être obtenu par un procédé de préparation de cyanhydrine d'acétone par transformation d'acétone avec de l'acide cyanhydrique.

11. Tétraméthylglycolide polymérisable pouvant être obtenu conformément à un procédé selon l'une des revendications 1 à 10.

12. Tétraméthylglycolide polymérisable selon la revendication 11, **caractérisé en ce que** le tétraméthylglycolide polymérisable contient en plus des produits secondaires, les produits secondaires comprenant
10 ppm en poids à 1000 ppm en poids d'acide 2-hydroxyisobutyrique,
10 ppm en poids à 1000 ppm en poids d'eau,
10 ppm en poids à 5000 ppm en poids de diacide 2-hydroxyisobutyrique, par rapport au poids total du tétraméthylglycolide polymérisable et
le rapport des sommes molaires de tous les protons azides des produits secondaires à la quantité molaire du tétraméthylglycolide polymérisable se situant dans la plage de 0,05 à 0,0001.

13. Tétraméthylglycolide polymérisable selon la revendication 12, **caractérisé en ce que** les produits secondaires comprennent en plus 1 ppm en poids à 1000 ppm en poids d'acide méthacrylique, par rapport au poids total du tétraméthylglycolide polymérisable.

14. Tétraméthylglycolide polymérisable selon la revendication 12 ou 13, **caractérisé en ce que** les produits secondaires comprennent en plus 1 ppm en poids à 5000 ppm en poids d'acide 2-hydroxyisobutyrique oligomère, par rapport au poids total du tétraméthylglycolide polymérisable.

15. Tétraméthylglycolide polymérisable selon l'une des revendications 12 à 14, **caractérisé en ce que** les produits secondaires comprennent en plus 1 ppm en poids à 500 ppm en poids d'au moins une dicétone de morpholine, qui est substituée par quatre groupes méthyle, par rapport au poids total du tétraméthylglycolide polymérisable.
